**Europäisches Patentamt**

**European Patent Office**

**Office européen des brevets**

(19)

(11) Veröffentlichungsnummer: **0 209 516**
**A2**

(12)

# EUROPÄISCHE PATENTANMELDUNG

(21) Anmeldenummer: 86890198.4

(22) Anmeldetag: 07.07.86

(51) Int. Cl.⁴: **A 61 F 2/36**, A 61 F 2/30

(30) Priorität: 16.07.85 AT 2097/85

(43) Veröffentlichungstag der Anmeldung: **21.01.87**
**Patentblatt 87/4**

(84) Benannte Vertragsstaaten: **BE CH DE FR GB IT LI LU NL SE**

(71) Anmelder: **VEREINIGTE EDELSTAHLWERKE AKTIENGESELLSCHAFT (VEW), Elisabethstrasse 12, A-1010 Wien (AT)**

(72) Erfinder: **Kaltenbrunner, Werner, Graschnitzstrasse 7, A-8641 St. Marein (AT)**
Erfinder: **Kotz, Rainer, Prof. Dr., Sonnenfelsgasse 11, A-1010 Wien (AT)**

(74) Vertreter: **Jellinek, Gerhard, Dr., Vereinigte Edelstahlwerke AG (VEW) Elisabethstrasse 12, A-1010 Wien (AT)**

(54) Knochenimplantat für Endoprothesen und Werkzeug zu dessen Einbringung.

(57) Die Erfindung betrifft ein Knochenimplantat für Endoprothesen, insbesondere für Gelenke bzw. Gelenksteile, vorzugsweise für solche des Bewegungs-Apparates des Menschens mit in eine Ausnehmung (40) eines Knochens (50), einbringbarem, von seinem der Diaphyse zugeordneten, distalen Ende (11) zum der Metaphyse zugeordneten, Ende (13) hin zunehmende Gesamtquerschnittsfläche aufweisendem Schaft (10), vorzugsweise mit im Bereich des metaphysären Endes (13), gegebenenfalls mit einstückig oder gesondert gebildetem Zwischenstück (20) angeordnetem bzw. anordenbarem Gelenk bzw. Gelenkteil (30), das im wesentlichen darin besteht, dass der im Knochen (50) bevorzugt primärstabil, verankerbare, insbesondere mit metallischem Werkstoff gebildete Schaft (10) mit einem längliche Gestalt aufweisenden, rotationssymmetrischen Körper mit, gegebenenfalls längsgekrümmte, bevorzugt jedoch im wesentlichen gerade Erzeugende aufweisendem Mantel, vorzugsweise mit im wesentlichen kegelstumpfförmigem Körper, gebildet ist, wobei die, bevorzugt bindemittelfrei, mit dem Knochen (50) in Kontakt bringbare Mantelfläche (100) eine Mehrzahl von im wesentlichen parallel zu Erzeugenden des Schaftkörpers (10), bevorzugt über dessen gesamte Länge (ℓ), sich erstreckenden, bevorzugt untereinander gleichartigen, Vorsprüngen (101) mit, vorzugsweise radial, von dessen Hauptachse (A) weg nach aussen hin weisenden, insbesondere etwa schneidenartigen, Kanten (102) aufweist.

Knochenimplantat für Endoprothesen
und  Werkzeug zu dessen Einbringung

Die Erfindung betrifft ein Knochenimplantat für Endoprothesen, insbesondere für Gelenke bzw. Gelenksteile,
vorzugsweise für solche des Bewegungs-, insbesondere
Gehapparates des Menschens mit in eine Ausnehmung eines
Knochens, vorzugsweise Röhrenknochens, einbringbarem,
von seinem der Diaphyse zugeordneten, einem Ende zum
der  Metaphyse zugeordneten, Ende hin zunehmende Gesamtquerschnittsfläche aufweisendem Schaft,  vorzugsweise mit  im  Bereich des metaphysären Endes, gegebenenfalls mit einstückig oder gesondert gebildetem Zwischenstück, angeordnetem bzw. anordenbarem Gelenk bzw. Gelenkteil.

Es ist eine große Zahl von derartigen implantierbaren
Prothesen, insbesondere für Hüftgelenke bekanntgeworden,
wobei für die im Knochen zu verankernden Schäfte verschiedene biokompatible, also mit Gewebe und Körperflüssigkeiten verträgliche und von diesen nicht korrodierbare Materialien, wie insbesondere Metalle, z.B.
hochlegierte  Stähle, Sonderlegierungen, Keramik, Faserverbund-Kunststoffe, Holz od. dgl. vorgeschlagen worden
sind. Bei aller Vielfalt der Ausbildung weisen die bekannten Verankerungsteile für solche Endoprothesen durchwegs einen länglichen Schaftkörper auf, welcher in den
Knochen eingebracht wird, wobei an dem proximalen Ende
des Schaftes entweder direkt ein Gelenkteil, z.B. eine
Gelenkspfanne, -fläche, -kugel oder -rolle angeordnet ist,
oder aber ein vom Schaft zum jeweiligen Gelenkteil, z.B.
zur Einhaltung bestimmter Lagewinkel und Abstände vorgesehener Verbindungs- oder Zwischenteil vorgesehen ist,
der häufig einstückig mit dem Schaft ausgebildet ist,
mit welchem der jeweilige Gelenkteil,bevorzugt lösbar verbindbar ist. Die Materialien von Gelenkteil und Prothesenschaft sind meist aufgrund der verschiedenen Funktionen nicht identisch. Die meisten der in längliche, insbesondere in Röhrenknochen einbringbaren Gelenks-Endoprothesenschäfte sind so ausgebildet, daß der Querschnitt vom proximalen zum distalen Endbereich hin abnimmt, womit eine Anpassung an die natürlichen Gegebenheiten, wie insbesondere die Form des Hohlraumes des Knochens ermöglicht ist, wodurch die Materialabtragung bei der Herstellung einer Ausnehmung zur Aufnahme des Implantatschaftes minimiert werden soll.

Zur Verankerung von Implantaten im Knochen sind einerseits der Einsatz von Bindemitteln, z.B. auf Kunststoffbasis, und andererseits eine bindemittelfrei arbeitende Technik der Verankerung bekanntgeworden, wobei bei Direktverankerung Probleme infolge Wärmeentwicklung und Gewebe-Unverträglichkeit des Bindemittels vermeidbar sind. Angestrebt wird, nach Einbringen des Schaftteiles möglichst ein längere Zeit beanspruchendes Einwachsenlassen desselben im Knochen nicht abwarten zu müssen und sofort nach Einbringung des Implantates eine stabile Verbindung von Schaft und Knochen zu schaffen.

Für die bisher handelsüblichen und in der Praxis verwendeten Implantatschafte, welche in der Regel keinen rotationssymmetrischen, sondern zur verdrehsicheren Verankerung z.B. etwa abgerundete Kanten aufweisenden mehr-, vorzugsweise etwa rechteckigen Querschnitt mit Längsnuten, Ausnehmungen, Öffnungen od. dgl. und zur Anpassung an den Knochen bei vielen Fabrikaten Längskrümmung aufweisen, erfolgt die Herstellung der Ausnehmung für deren Aufnahme im wesentlichen in der Weise, daß eine solche mittels einer der Gestalt des Schaftes im wesentlichen entsprechend geformten Raspel, Räumwerkzeug od. dgl., die mittels z.B. pneumatischen Antriebs oszillierend bewegt wird, in den Knochen gegen die Diaphyse hin vorgetrieben wird. Obwohl diese Technik schon lange Zeit gebräuchlich ist, lassen sich damit Ausnehmungen, deren Wandung über große Bereiche oder gänzlich mit der Außenfläche des Implantatschaftes präzise in satt anliegendem Kontakt kommen können, nicht erreichen. Vielmehr liegen diese bekannten verschiedene Formen aufweisenden Implantatschafte nur an wenigen Stellen und über relativ kleine Bereiche an der Ausnehmungs-Wandung an, womit jedoch gerade an diesen Stellen die Einleitung der mechanischen Kräfte über Zonen relativ geringer Flächenausdehnung und damit hohe Druckbelastung erfolgt. Solche Druckspitzen jedoch bewirken, wie bekannt, eine Störung des biologischen Gleichgewichtes im Knochen, auf welche dieser in der Weise reagiert, daß gerade an diesen Stellen erhöhter Druckbeanspruchung ein Abbau der Knochensubstanz und damit eine Schwächung der Cortex und somit die Gefahr einer Lockerung des Sitzes des Schaftes eintritt. Bekanntgeworden ist weiters z.B. aus der DE-OS 2 049 111 ein Protheseglied mit kurzkegeligem Implantatschaft und im wesentlichen glatter Oberfläche, wobei gemäß dieser Druckschrift mittels eines eigenen Mechanismus eine Anpassung des Elastizitätsmeduls des Schaftes an jenen des Knochens erreicht werden soll. Eine auf längere Zeit beanspruchbare lockerungs- und verdrehsichere Verankerung im Knochen ist bei der dort gezeigten Ausführungsform nicht erreichbar.

Die vorliegende Erfindung hat sich die Aufgabe gestellt, ein Knochenimplantat für Endoprothesen für Gelenke zu schaffen, bei welchem eine vereinfachte und wesentlich präzisere Technik der Einbringung der Ausnehmung zur Aufnahme des Implantates im Knochen mit einer Vermeidung von Druckspitzen durch gleichmäßige Einleitung der mechanischen Belastung und Kräfte in den Knochen über größtmögliche von vornherein satt anliegende Kontaktflächen kombiniert werden kann, wobei gleichzeitig höchste Sicherheit gegen Axialverdrehung und weiters ausgezeichnete physiologische Verträglichkeit und hohe Eignung für Langzeiteinsatz gegeben sind.

Gegenstand der Erfindung ist somit ein Knochenimplantat der eingangs genannten Art, das im wesentlichen darin besteht, daß der im Knochen, bevorzugt primärstabil, verankerbare, insbesondere mit metallischem Werkstoff gebildete Schaft mit einem längliche Gestalt aufweisenden rotationssymmetrischen Körper mit, gegebenenfalls längsgekrümmte, bevorzugt jedoch im wesentlichen gerade Erzeugende aufweisendem Mantel, vorzugsweise mit im wesentlichen kegelstumpfförmigem Körper, gebildet ist, wobei die, bevorzugt bindemittel-frei, mit dem Knochen in Kontakt bringbare Mantelfläche eine Mehrzahl von im wesentlichen parallel zu Erzeugenden des Schaftkörpers, bevorzugt im wesentlichen über dessen gesamte Länge, sich erstreckenden, bevorzugt untereinander gleichartigen Vorsprüngen mit im wesentlichen, vorzugsweise radial, von dessen Hauptachse weg nach außen hin weisenden, vorzugsweise etwa schneidenartigen, Kanten aufweist. Durch die rotationssymmetrische, zumindest im wesentlichen konische Gestaltung des Schaftkörpers wird eine wesentliche Vereinfachung der Herstellung der Ausnehmung zu dessen Einbringung in den Knochen erreicht, wobei diese Ausnehmung hinsichtlich Lage und Tiefe hochpräzise mit einem der jeweiligen Schaftdimension entsprechenden Schneid- bzw. Fräswerkzeug ohne oszillierendes Schaben od. dgl. ausgeführt werden kann. Damit ist, da hohe Übereinstimmung zwischen Konizität der Ausnehmungswand und der Außenmantelfläche des Schaftkörpers erzielbar ist, grundsätzlich wesentlich vergrößerte Kontaktfläche zum Knochen gegeben. Überraschend war, daß dabei die Kanten aufweisenden Vorsprünge einen satten Sitz, des Schaftes im Knochen mit hohem Flächenkontakt nicht behindern, dabei aber höchste Sicherheit gegen ein Verdrehen zu gewährleisten imstande sind. Die Kanten verdrängen die Knochensubstanz beim Einschlagen des Schaftes seitlich, fressen sich geringfügig in die Cortex ein, wobei sich in kurzer Zeit der biologische Gleichgewichtszustand wieder einstellen kann. Wie sich zeigte, ist weiters jegliche Gefahr eines keilartigen Aufsprengens des Knochens, wie es z.B. bei flächiger Ausbildung von Verankerungselementen zu beobachten ist, völlig vermieden. Durch die rotationssymmetrisch konische Ausbildung ist zentrierendes Herausarbeiten der Ausnehmung und selbstzentrierendes Einbringen des Schaftes in den Knochen sichergestellt. Bei dieser Ausbildung ist immer an praktisch allen Bereichen des Schaftes selbst bei Nachsetzvorgängen, die, wie im übrigen gefunden wurde, höchstens im mm-Bereich stattfinden, immer ein sattes Anliegen der Kontaktflächen des Schaftes am Knochen gegeben. Die im wesentlichen in Richtung der Knochenerstreckung wirkenden Kräfte werden mittels des Konus auf relativ großer Fläche schräg mit jeweils geringerer Quer-Vektoren in den Knochen eingeleitet, sodaß Druckbelastungsspitzen mit der Gefahr eines lokalen Abbaues von Knochensubstanz in den mechanisch höher belasteten Zonen des Kontaktes der Prothese mit dem Knochen vermieden sind. Weiters ist, wie sich zeigte, bei der erfindungsgemäßen Ausbildung der Kontaktfläche auch hohe Sicherung gegen unerwünschtes Herausziehen des Schaftes aus dem Knochen gewährleistet. Durch die Kanten der Vorsprünge kann darüber hinaus der Einwachsvorgang stimuliert werden, wobei

an sich sofort nach Einbringung des Implantates ohne Abwarten eines Einwachsens dessen primärstabile Verankerung gegeben ist. Durch die Anordnung einer Vielzahl längsverlaufender Vorsprünge ist weiters im Bereich der Nutgründe dazwischen ein Längstransport und -austausch biologischen Fluids an der Kontaktfläche und damit die Einstellung und Aufrechterhaltung des biologischen Gleichgewichtes auch in Längsrichtung sicherstellbar. Der trotz bzw. mit Hilfe der Kanten der Vorsprünge gleich nach dem Einschlagen erreichbare Vollflächenkontakt ist imstande, auch bei starken Querbelastungen mit Hebelwirkung ein Lockern der Prothese vollkommen hintanzuhalten. Betont sei ferner, daß das neue Implantat, wenn auch voraussichtlich für den Teilersatz von menschlichen Oberschenkelknochen am meisten zum Einsatz kommend, durchaus, da im wesentlichen über die Konusdimension anpaßbar z.B. für Kniegelenke, aber auch für andere Gelenke des Menschens, z.B. Ellbogen- oder Schultergelenke, geeignet ist, wobei sich diese Anwendungspalette, insbesondere als Folge der überraschend hohen Sicherheit des neuen Prothesenschaftes gegen Herausziehen anbietet.

Insbesondere im Hinblick auf den Knocheninnenbau und damit auf die Langzeit-Stabilität und -Festigkeit der Implantat/Knochen-Verbindung sowie weiters zur Minimierung der zum fixen Verankern bei Herstellung der Implantat-Ausnehmung abzutragenden Knochensubstanz-Menge ist es bevorzugt, wenn die Konizität der Knochenkontaktfläche bzw. Neigung der Kanten der Vorsprünge des Schaftkörpers im wesentlichen einem Verhältnis der Differenz von dessen Durchmesser jeweils am proximalen und am distalen Ende des Schaftkörpers zu dessen Länge von 1 : 4 bis 1 : 20, insbesondere von 1 : 6 bis 1 : 15, bevorzugt von 1 : 8 bis 1 : 12, entspricht. Bei Einhalten dieser Konizität wird ein besonders ausgeglichenes Verhältnis zwischen radial wirksamen Kräften und Aufnahmekapazität des Knochens dafür erzielt, und es kann der notwendige Abtrag von Knochensubstanz bei der Implantat-Ausnehmung gering gehalten werden. In der Praxis hat sich bei Oberschenkelknochen-Implantaten ein wie oben genanntes Verhältnis im Bereich von etwa 1 : 10 besonders bewährt.

Für die Physiologie des Knochens und weiters zur Erhöhung der Sicherheit gegen Verdrehbarkeit um die Schaftlängsachse und gegen unbeabsichtigte Extraktion des Implantates hat es sich als vorteilhaft erwiesen, wenn das Verhältnis des Abstandes von jeweils zwei einander benachbarten Vorsprüngen, gegebenenfalls Kanten, voneinander zum Durchmesser des Schaftkörpers, jeweils in dessen Mitte etwa 1 : 5 bis 1 : 30, vorzugsweise etwa 1 : 10 bis 1 : 20, insbesondere etwa 1 : 10 bis 1: 15, beträgt. Für die Praxis bedeutet dies, daß etwa ein Oberschenkelgelenks-Endoprothesenschaft für einen Erwachsenen, z.B. einen proximalen Konus-Durchmesser von 20 bis 30 mm und einen distalen Konus-Durchmesser von 10 bis 15 mm bei einer Länge von 100 bis 160 mm aufweisen kann und der Abstand zweier benachbarter Vorsprünge voneinander am proximalen, also breiten Schaft-Ende im Bereich von etwa 0,7 bis 1,1 mm betragen kann.

Für die Versorgung des Knochens in Implantatnähe und den Substanz-Austausch in Längsrichtung sowie für die Verdrehsicherung der Verankerung vorteilhaft ist es, wenn dafür gesorgt ist, daß das Verhältnis der Höhe der Vorsprünge zum Durchmesser des Schaftkörpers, jeweils in dessen Mitte etwa 1 : 5 bis 1 : 30, insbesondere etwa 1 : 10 bis 1 : 20, beträgt.

Insbesondere zur Sicherstellung der von vornherein, also sofort ab Einbringen der Prothese primärstabilen Konussteckverbindung, bei der anfänglich ein Eindringen der Kanten in die Knochensubstanz unter deren geringfügiger Verdrängung erfolgt, günstig erwiesen hat es sich, wenn die von der Hauptachse weg nach außen weisenden Kanten der Vorsprünge im wesentlichen einen Kantenradius von etwa 10 bis 100 μm, insbesondere von etwa 20 bis 50 μm, aufweisen.

Sowohl für die Technik bei der Operation, wie für die postoperative Einheilung der Prothese hat es sich als besonders vorteilhaft herausgestellt, wenn vorgesehen ist, daß die Vorsprünge zumindest im Nahbereich ihrer Kanten im wesentlichen, gegebenenfalls gleichschenkelig, dreieckig mit einem Kantenwinkel $\alpha$ von 30 bis 150°, insbesondere von 60 bis 120°, vorzugsweise im Bereich von etwa 90°, ausgebildet sind, wobei auch die Fertigung keines besonderen Aufwandes bedarf.

Wenn, wie weiters in günstiger Weise vorgesehen, die Bereiche, insbesondere Vertiefungen zwischen den Vorsprüngen gerundet, vorzugsweise mit größerem Rundungsradius als deren Kanten ausgebildet sind, können etwa Verstopfungen der Längsnuten, z.B. durch steckenbleibende Knochensubstanzteilchen, die z.B. von der Operation herrühren, verhindert werden.

Fertigungstechnisch, jedoch auch hinsichtlich der Kräfte-Einleitung und -Verteilung kann es weiters günstig sein, wenn die Höhe der Vorsprünge vom proximalen zum distalen Ende des Schaftkörpers hin abnimmt.

Um sofort bei Einbringung ganz satten Sitz des Implantates im Knochen zu erreichen und die Flächen-Belastung besonders gleichmäßig und gering zu halten, kann es weiters von Vorteil sein, wenn die Höhe der Vorsprünge vom proximalen zum distalen Ende des Schaftkörpers hin im wesentlichen konstant ist, und der Schaftkörper eine nur dem Schaftdurchmesser im wesentlichen an seinem distalen Ende entsprechende Anzahl durchgehender Vorsprünge aufweist, zwischen welchen zum proximalen Ende hin erst beginnende, zu demselben hin sich kürzer erstreckende, weitere, vorzugsweise gleichartige, Vorsprünge angeordnet sind.

Allerdings erfordert diese Ausführungsform bei der Fertigung etwas mehr Aufwand. Für das Einbringen dieses Implantats kann zuerst in den Knochen die rotationssymmetrische kegelstumpfförmige Ausnehmung eingebracht werden, wonach dann zur Erhöhung der Präzision und Primärstabilität mit einem oszillierenden Abtrage-Werkzeug, also etwa mit einer entsprechend den verschiedenen Kanten mit Schneiden versehenen Raspel zur Positionierung der Vorsprünge entsprechende Längsriefen entsprechender Länge in die Konus-Wandung der Ausnehmung im Knochen eingebracht werden können.

Wenn, wie gemäß einer weiteren Variante vorteilhaft vorgesehen, der Schaftkörper an seinem distalen Ende einen Abschnitt mit im wesentlichen, gegebenenfalls schief-, kegelstumpfförmiger Mantelfläche mit einem größeren Öffnungswinkel ihrer Erzeugenden als jenem des Schaftkörpers selbst aufweist, können ein seitliches Eindringen der distalen Kante des Schaftes bei nicht ganz korrekt eingebrachtem Implantat in die Cortex und damit eine abrupte Schwächung vermieden werden, und es ist im distalen Bereich an der Peripherie rundum ein stetiger Übergang von Implantat zum Knochen ohne horizontale Kernstellen sichergestellt.

Bevorzugt weist der Schaftkörper an seinem proximalen Ende einen im wesentlichen zylindrischen Fortsatz, gegebenenfalls mit Verankerungselement, z.B. Innengewinde, auf. Bei einem derartigen proximalen Ansatz können eine unnötige Abtragung von Spongia in der Metaphyse und ein seitliches Abdrängen des Schaftkopfes vermieden werden. Das Verankerungselement kann ein späteres operatives Entfernen des Implantates wesentlich erleichtern.

Wenn, wie weiters vorteilhaft vorgesehen, der Schaftkörper einen zumindest zu seinem distalen Ende hin offenen, vorzugsweise zylindrischen, Hohlraum aufweist, können Gewichtsersparnis und verbesserte Versorgung des Gewebes infolge des innen freien Längsflusses der Körperfluide sichergestellt werden.

Dieser Fluß bzw. auch das Einwachsen des Implantates können noch weiter gefördert werden, wenn, wie bevorzugt, der Schaftkörper im wesentlichen radiale Verbindungsöffnungen zwischen Hohlraum und Knochenkontaktfläche aufweist.

Eine Kerbwirkungs-Verminderung bzw. eine Art rundum elastischer distaler Endbereich bei Hohlraum aufweisenden Implantatschaft nach Art einer Ringlippe beim Sitz im Knochen und damit Herabsetzung und verbesserte Verteilung der Druckbelastungen z.B. bei hebelartiger Beanspruchung lassen sich vorteilhaft erreichen, wenn der Übergang von der Knochenkontaktfläche zur distalen Endfläche insbesondere zum Hohlraum, des Schaftkörpers im wesentlichen gerundet, vorzugsweise mit einem Radius von etwa 0,2 bis 0,8 mm, insbesondere von etwa 0,5 mm, ausgebildet ist.

Wenn, wie gemäß einer weiteren bevorzugten Form die Knochenkontaktfläche, vorzugsweise vollumfänglich, umlaufende, bevorzugt kantengerundete, entlang Ebenen im wesentlichen senkrecht zur Hauptachse verlaufende, gegebenenfalls gerundete Nutgründe aufweisende, Nuten bzw. Rillen aufweist, sind besonders hohe Verankerungssicherheit bei Zugbeanspruchung sowie Förderung des horizontal-peripheren Austausches der Körperfluide und Möglichkeit des Umwachsens gegeben.

Bevorzugt ist die Anzahl der umlaufenden Nuten bzw. Rillen begrenzt, wobei eine Zahl von unter 10, vorzugsweise von unter 5, als ausreichend anzusehen ist.

Im Sinne besonders guter Verankerung und geförderten peripheren Fluid-Austausches hat es sich weiters als günstig erwiesen, wenn die Tiefe der umlaufenden Nuten, jeweils an der Stelle ihres Vorhandenseins zumindest gleich der dortigen Höhe der längsverlaufenden Vorsprünge gehalten ist.

Wenn, wie weiters vorteilhaft vorgesehen, der Körper des Schaftes mit einem Zwischenstück für die Anordnung eines Gelenkes bzw. Gelenkteiles einstückig aus metallischem Werkstoff gefertigt, insbesondere geschmiedet ist, ist bei fertigungstechnischer Einfachheit hohe Festigkeit der Verbindung des Implantatschaftes mit einem von ihm z.B. seitab ausgehenden, z.B. für die Aufnahme einer beschichteten Gelenkskugel od. dgl. geeigneten Zwischen- bzw. Halsstück erreichbar, da mit dem Schmieden kontinuierlicher Verlauf der Werkstoff-Fasern vom Schaft in das Zwischenstück, welche eine glatte Einleitung der Kräfte in den Implantatschaft und in den Knochen sicherstellt, erreicht werden kann.

Gegenstand der Erfindung ist schließlich weiters ein Werkzeug zur Vorbereitung des Knochens zur vorzugsweise bindemittelfreien Einbringung des erfindungsgemäßen Knochenimplantates für Endoprothesen wie vorher beschrieben, das im wesentlichen gekennzeichnet ist durch einen vorzugsweise pneumatisch, betreibbar drehbaren, an seiner Außenseite, eine, vorzugsweise ungerade, Anzahl im wesentlichen schraubig verlaufender Schneiden aufweisenden, insbesondere hinsichtlich Konizität, im wesentlichen dem Schaft eines jeweils in einen Knochen einzubringenden Knochenimplantates entsprechenden, im wesentlichen kegelförmigen Körper, insbesondere Fräskörper mit an seinem distalen Ende einen diaphysär weiter als später der Implantatkörper in den Knochen eindringbarem die Schneiden aufweisendem Fortsatz. Durch diese Ausbildung des Werkzeuges wird besonders gut lagesteuerbarer runder Lauf innerhalb des harten Knochens bei äußerst kurzer Zeitdauer der Einbringung der Ausnehmung für die folgende Aufnahme des Implantates erreicht, wobei später eine stufenartige Einkerbung des Knochens mit dem distalen Schaftende im wesentlichen vermeidbar ist, da infolge des genannten Fortsatzes die Ausnehmung mit identischer Konizität etwas weiter in den Knochen hinein vorgetrieben wird, als der schließlich in die Ausnehmung eingeschlagene Implantatschaft.

Wenn günstigerweise die Schneiden um den Querschnitt des Werkzeugkörpers asymmetrisch, insbesondere z.B. 0,5 bis 3$^o$ unterschiedliche Winkel einschließend, verteilt sind.

Wenn, wie weiters bevorzugt vorgesehen, bei dem Werkzeug das distale Ende des Werkzeug-, insbesondere Fräskörpers bzw. dessen Fortsatz, vorzugsweise der dortige Bereich der Schneiden, im wesentlichen abgerundet ausgebildet ist, sind besonders gut zentrierender Lauf des Werkzeuges und damit hohe Präzision und Anpassung der Ausnehmung im Knochen an die Form des Endoprothesenschaftes sichergestellt.

Schließlich ist es bei dem Werkzeug von Vorteil, wenn es metaphysär einen, vorzugsweise die Schneiden des Fräskörpers fortsetzende, Schneiden aufweisenden, zylindrischen Fortsatz aufweist, wodurch unnötiger Substanzabtrag der Metaphyse bei Herstellung der Implantatausnehmung vermeidbar ist.

Anhand der in der Zeichnung dargestellten Beispiele wird die Erfindung näher erläutert, wobei die Fig. 1 bis 1c eine Seitenansicht und Detaildarstellungen eines erfindungsgemäßen Knochenimplantates für den Ersatz eines Oberschenkelgelenkes, die Fig. 2 eine Schrägansicht einer speziellen Ausführungsform des Schaftkörpers eines erfindungsgemäßen Implantates und die Fig. 3 eine Seitenansicht eines erfindungsgemäßen bevorzugt einsetzbaren Fräswerkzeuges zur Einbringung einer Ausnehmung in den Knochen zur Aufnahme eines Implantates gemäß der Erfindung zeigen.

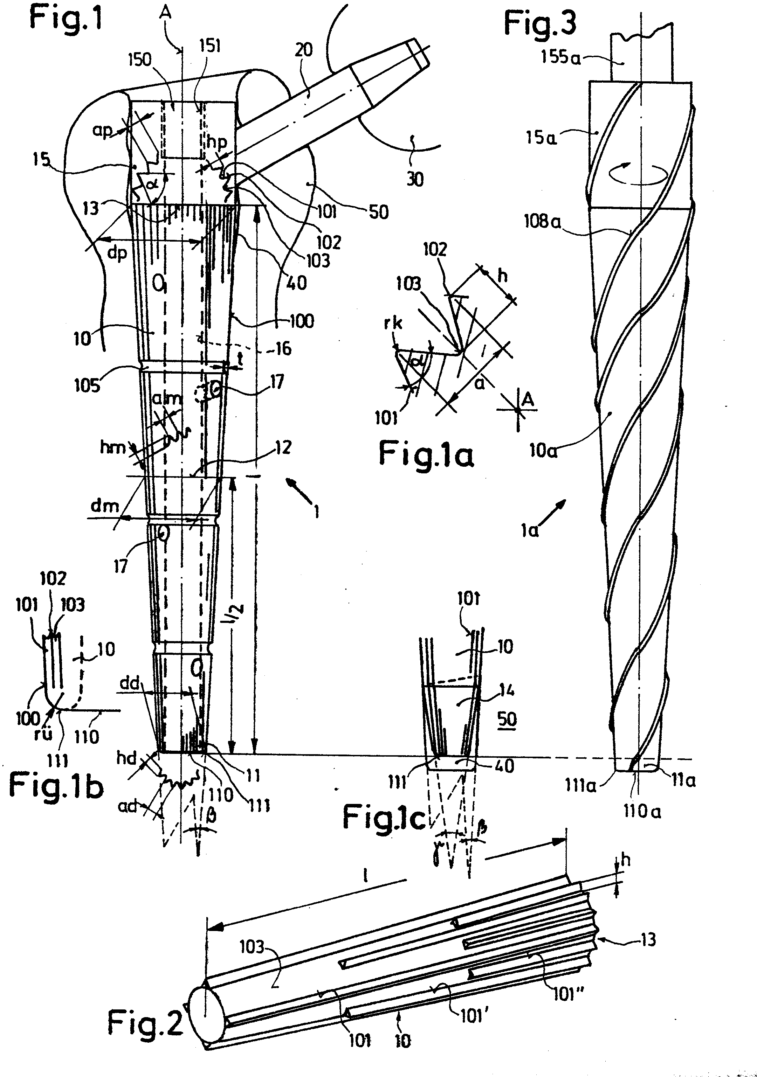

Das Implantat 1 gemäß Fig. 1 weist einen gerade Erzeugende aufweisenden länglich kegelsstumpfförmigen Schaft 10 mit einem seitlich schräg angesetzten Zwischenstück 20 für die Anordnung einer Gelenkskugel 30 auf, wobei die mit dem Knochen 50 in dessen Ausnehmung 40 im implantierten Zustand in Berührung stehende Kontaktfläche 100 des Implantatschaftes 10 über im wesentlichen seine gesamte Länge $\ell$ sich erstreckende Vorsprünge 101 mit, siehe Fig. 1a, radial von der Schaftachse A weg nach außen weisenden Kanten 102 mit einem Kantenwinkel $\alpha$, von hier

0209516

z.B. von etwa 75°, aufweist. Die Zwischenräume 103 zwischen den Vorsprüngen 101 sind im Nutgrund gerundet ausgebildet, die Kanten 102 weisen, wie das Detail der Fig. 1a zeigt, eine schneidenartige Kante mit einem Radius rk, z.B. im Bereich zwischen 10 und 100 µm auf. Ihr Abstand a, z.B. Schaftmitte 12 : 0,8 mm, der am proximalen Schaftende 13 ap, in der Schaftmitte 12 am und am distalen Schaftende 11 ad beträgt, sowie ihre Höhe h, die entsprechend hp, hm, hd beträgt, nimmt vom proximalen 13 zum distalen Ende 11 hin ab. Die Konizität des Schaftkörpers 10 ist durch das Verhältnis der Differenz der Schaftdurchmeser dd am distalen 11 (dm in der Schaftmitte 12) und dp am proximalen Ende 13 zur Gesamtlänge $\ell$ gegeben und beträgt bevorzugt etwa 1 : 10. Außer den Längsvorsprüngen 101 kann der Schaftkörper auch rundum laufende Nuten 105 aufweisen, deren Tiefe t größer sein kann als die Höhe h der Vorsprünge 101. Am proximalen Ende 13 geht der Schaft 10 hier in einen glatten zylindrischen Fortsatz 15 über, welcher eine Ausnehmung 150 mit Innengewinde 151 aufweist, welches die Verankerung eines Extraktions-Werkzeuges für den Fall eines operativen Entfernens des Implantates 1 aus dem Knochen 50 erleichtert. Der gezeigte Implantatschaft 10 kann, wie mit unterbrochener Linie angedeutet, weiters einen, hier zylindrischen, Hohlraum 16 aufweisen, wobei der aus dem Detail der Fig. 1b ersichtliche Radius rü des Überganges 111 von der Kontaktfläche 100 zum hier zur Ausnehmung 150 durchgehenden Hohlraum 16 im wesentlichen gleich sein kann wie zu einer distalen Stirnfläche 110, wenn kein Hohlraum in Schaft 10 vorgesehen ist. Vom Hohlraum 16 können, in Fig. 1 punktiert angedeutet, Öffnungen 17 zur Kontaktfläche 100 ausgehen.

Aus Fig. 1c ist ersichtlich, wie gegebenenfalls das distale Ende 11 des Schaftkörpers 10 einen Konus 14 mit einem größeren Konusöffnungswinkel $\gamma$ aufweisen kann, als jener $\beta$ des Schaftes 10 selbst ist, wobei der Konus 14, wie strichliert angedeutet, auch schiefkegelig sein kann. Der Konus 14 kann ein örtlich einseitiges Einkerben der Cortex durch die distale Schaftkante 111 beim Einschlagen mit erhöhter Sicherheit vermeiden helfen.

Der Implantat-Schaftkörper 10, wie ihn die Fig. 2 nur schematisch prinzipiell zeigt, unterscheidet sich bei im wesentlichen gleicher Grundbauart von jenem gemäß Fig. 1 im wesentlichen dadurch, daß er durchgehende Vorsprünge 101 aufweist, deren Höhe h jedoch über die Schaftlänge $\ell$ im wesentlichen konstant ist. Gegen das proximale Ende 13 hin sind in den breiter werdenden Nuten 103 zu diesem Ende 13 sich erstreckende Zwischen-Vorsprünge 101' und noch weiter in dieser Richtung beginnen dann weitere Zwischen-Vorsprünge 101", wobei bevorzugt alle Vorsprünge 101, 101', 101" gleichartig ausgebildet sind.

Das Werkzeug 1a der Fig. 3 zur Einbringung einer Ausnehmung für die Aufnahme eines Implantatschaftes, z.B. gemäß Fig. 1 weist einen Fräserkörper 10a mit im wesentlichen gleicher kegelstumpfförmiger Kontur wie der Implantatschaft 10 (siehe Fig. 1) auf. Er ist im Vergleich zu diesem im Konus mit einem Fortsatz 11a etwas verlängert ausgebildet, der zur distalen Stirnseite 110a hin einen in der Kontur abgerundeten Übergang 111a aufweist. Proximal besitzt der Fräskörper einen dem Ansatz 15 des Schaftes 10 der Fig. 1 entsprechenden zylindrischen Ansatz 15a, welcher einen Fortsatz 155a mit nicht gezeigtem Verbindungselement zum Verbinden mit einen Drehantrieb aufweist. Über den Ansatz 15a, den Konus 10a und dessen distalen Verlängerungsfortsatz 11a erstrecken sich mit etwa steilschraubigem Verlauf, bevorzugt in ungerader Anzahl, z.B. sieben, hier durchgehende, Schneiden 108a. Die durchgehenden Schneiden sind bezogen auf den Querschnitt des Werkzeuges asymmetrisch verteilt, d.h. sie schließen untereinander jeweils verschiedene Winkel ein. Weiters ist die Richtung der Spirale entgegengesetzt der Drehrichtung, da sonst das Fräswerkzeug in den Knochen geschraubt wird.

Ein wie hier gezeigtes Fräswerkzeug 1a ermöglicht hohe Präzision bei der Herstellung der Ausnehmung im Knochen zur Aufnahme eines Implantates, wobei die Zeit dieser Herstellung wesentlich herabgesetzt wird.

Patentansprüche :

1. Knochenimplantat für Endoprothesen, insbesondere für Gelenke bzw. Gelenksteile, vorzugsweise für solche des Bewegungs-, insbesondere Geh-Apparates des Menschens mit in eine Ausnehmung (40) eines Knochens (50), vorzugsweise Röhrenknochens, einbringbarem, von seinem der Diaphyse zugeordneten, distalen Ende (11) zum der Metaphyse zugeordneten, Ende (13) hin zunehmende Gesamtquerschnittsfläche aufweisendem Schaft (10), vorzugsweise mit im Bereich des metaphysären Endes (13), gegebenenfalls mit einstückig oder gesondert gebildetem Zwischenstück (20) angeordnetem bzw. anordenbarem Gelenk bzw. Gelenkteil (30), dadurch gekennzeichnet, daß der im Knochen (50) bevorzugt primärstabil, verankerbare, insbesondere mit metallischem Werkstoff gebildete, Schaft (10) mit einem längliche Gestalt aufweisenden, rotationssymmetrischen Körper mit, gegebenenfalls längsgekrümmte, bevorzugt jedoch im wesentlichen gerade Erzeugende aufweisendem Mantel, vorzugsweise mit im wesentlichen kegelstumpfförmigem Körper, gebildet ist, wobei die, bevorzugt bindemittel-frei, mit dem Knochen (50) in Kontakt bringbare Mantelfläche (100) eine Mehrzahl von im wesentlichen parallel zu Erzeugenden des Schaftkörpers (10), bevorzugt im wesentlichen über dessen gesamte Länge ($\ell$), sich erstreckenden, bevorzugt untereinander gleichartigen, Vorsprüngen (101) mit im wesentlichen, vorzugsweise radial, von dessen Hauptachse (A) weg nach außen hin weisenden, insbesondere etwa schneidenartigen, Kanten (102) aufweist.

2. Knochenimplantat nach Anspruch 1, dadurch gekennzeichnet, daß die Konizität der Knochenkontaktfläche bzw. Neigung der Kanten (102) der Vorsprünge (101) des Schaftkörpers (10) im wesentlichen einem Verhältnis der Differenz von dessen Durchmessern(dp-dd) jeweils am proximalen (13) und am distalen (11) Ende des Schaftkörpers (10) zu dessen Länge ($\ell$) von 1 : 4 bis 1 : 20, insbesondere von 1 : 6 bis 1 : 15, bevorzugt von 1 : 8 bis 1 : 12, entspricht.

3. Knochenimplantat nach Anspruch 1 oder 2, dadurch gekennzeichnet, daß das Verhältnis des Abstandes (am) von jeweils zwei einander benachbarten Vorsprüngen (101), gegebenenfalls Kanten (102), voneinander zum Durchmesser (dm) des Schaftkörpers (10), jeweils in dessen Mitte (12) etwa 1 : 5 bis 1 : 30, vorzugsweise etwa 1 : 10 bis 1 : 20, insbesondere etwa 1 : 10 bis 1 : 15 beträgt.

4. Knochenimplantat nach einem der Ansprüche 1 bis 3, dadurch gekennzeichnet, daß das Verhältnis der Höhe (hm) der Vorsprünge (101) zum Durchmesser (dm) des Schaftkörpers (10), jeweils in dessen Mitte (12) etwa 1 : 5 bis 1 : 30, insbesondere etwa 1 : 10 bis 1 : 20, beträgt.

5. Knochenimplantat nach einem der Ansprüche 1 bis 4, dadurch gekennzeichnet, daß die von der Hauptachse (A) weg nach außen weisenden Kanten (102) der Vorsprünge (101) im wesentlichen einen Kantenradius (rk) von etwa 10 bis 100 $\mu$m, insbesondere von etwa 20 bis 50 $\mu$m, aufweisen.

6. Knochenimplantat nach einem der Ansprüche 1 bis 5, dadurch gekennzeichnet, daß die Vorsprünge (101) zumindest im Nahbereich ihrer Kanten (102) im wesentlichen, gegebenenfalls gleichschenkelig, dreieckig mit einem Kantenwinkel $\alpha$ von 30 bis 150°, insbesondere von 60 bis 120°, vorzugsweise im Bereich von etwa 90°, ausgebildet sind.

7. Knochenimplantat nach einem der Ansprüche 1 bis 6, dadurch gekennzeichnet, daß die Bereiche, insbesondere Vertiefungen (103), zwischen den Vorsprüngen (101) gerundet, vorzugsweise mit größerem Rundungsradius als die Kanten (102), ausgebildet sind.

8. Knochenimplantat nach einem der Ansprüche 1 bis 7, dadurch gekennzeichnet, daß die Höhe (h) der Vorsprünge (101) vom proximalen (13) zum distalen (11) Ende des Schaftkörpers (10) hin abnimmt.

9. Knochenimplantat nach einem der Ansprüche 1 bis 7, dadurch gekennzeichnet, daß die Höhe (h) der Vorsprünge (101) vom proximalen (13) zum distalen (11) Ende des Schaftkörpers (10) hin im wesentlichen konstant ist und der Schaftkörper (10) eine nur dem Schaftdurchmesser (dd) im wesentlichen an seinem distalen Ende (11) entsprechende Anzahl durchgehender Vorsprünge (101) aufweist, zwischen welchen zum proximalen Ende (13) hin erst beginnende, zu demselben hin sich kürzer erstreckende, weitere, vorzugsweise gleichartige, Vorsprünge (101', 101") angeordnet sind.

10. Knochenimplantat nach einem der Ansprüche 1 bis 9, dadurch gekennezeichnet, daß der Schaftkörper (10) an seinem distalen Ende (11) einen Abschnitt (14) mit im wesentlichen, gegebenenfalls schief-, kegelstumpfförmiger Mantelfläche mit einem größeren Öffnungswinkel ($\gamma$) ihrer Erzeugenden als jenem ($\beta$) des Schaftkörpers (10) selbst aufweist.

11. Knochenimplantat nach einem der Ansprüche 1 bis 10, dadurch gekennzeichnet, daß der Schaftkörper (10) an seinem proximalen Ende (13) einen im wesentlichen zylindrischen Fortsatz (15), gegebenenfalls mit Verankerungselement, z.B. Innengewinde (151), aufweist.

12. Knochenimplantat nach einem der Ansprüche 1 bis 11, dadurch gekennzeichnet, daß der Schaftkörper (10) zumindest einen, zumindest zu seinem distalen Ende (11) hin offenen, vorzugsweise zylindrischen, Hohlraum (16) aufweist.

13. Knochenimplantat nach einem der Ansprüche 1 bis 12, dadurch gekennzeichnet, daß der Schaftkörper (10) im wesentlichen radiale Verbindungsöffnungen (17) zwischen Hohlraum (16) und Knochenkontaktfläche (100) aufweist.

14. Knochenimplantat nach einem der Ansprüche 1 bis 13, dadurch gekennzeichnet, daß der Übergang (111) von der Knochenkontaktfläche (100) zur distalen Endfläche (110), insbesondere zum Hohlraum (16), des Schaftkörpers (10) im wesentlichen gerundet, vorzugsweise mit einem Radius (rü) von etwa 0,2 bis 0,8 mm, insbesondere von etwa 0,5 mm, ausgebildet ist.

15. Knochenimplantat nach einem der Ansprüche 1 bis 13, dadurch gekennzeichnet, daß die Knochenkontaktfläche (100), vorzugsweise vollumfänglich umlaufende, bevorzugt kantengerundete, entlang Ebenen im wesentlichen senkrecht zur Hauptachse (A) verlaufende, gegebenenfalls gerundete Nutgründe aufweisende, Nuten bzw. Rillen (105) aufweist.

16. Knochenimplantat nach einem der Ansprüche 1 bis 15, dadurch gekennzeichnet, daß die Anzahl der umlaufenden Nuten bzw. Rillen (105) unter 10, vorzugsweise unter 5, beträgt.

17. Knochenimplantat nach einem der Ansprüche 1 bis 16, dadurch gekennzeichnet, daß die Tiefe (t) der umlaufenden Nuten (105) jeweils an der Stelle ihres Vorhandenseins zumindest gleich der dortigen Höhe (h) der längsverlaufenden Vorsprünge (101) gehalten ist.

18. Knochenimplantat nach einem der Ansprüche 1 bis 17, dadurch gekennzeichnet, daß der Körper des Schaftes (10) mit einem Zwischenstück (20) für die Anordnung eines Gelenkes bzw. Gelenkteiles (30) einstückig aus metallischem Werkstoff gefertigt, insbesondere geschmiedet, ist.

19. Werkzeug zur Vorbereitung des Knochens zur, vorzugsweise bindemittelfreien, Einbringung des Knochenimplantates für Endoprothesen nach einem der Ansprüche 1 bis 18, gekennzeichnet durch einen, vorzugsweise pneumatisch betreibbar drehbaren, an seiner Außenseite, eine, vorzugsweise ungerade, Anzahl im wesentlichen schraubig verlaufender Schneiden (108a) aufweisenden, insbesondere hinsichtlich Konizität, im wesentlichen dem Schaft (10) eines jeweils in einen Knochen (50) einzubringenden Knochenimplantates (1) entsprechenden, im wesentlichen kegelförmigen Körper (10a), insbesondere Fräskörper mit an seinem distalen Ende einen diaphysär weiter als später der Implantatkörper (10) in den Knochen (50) eindringbarem, die Schneiden (108a) aufweisendem Fortsatz (11a).

20. Werkzeug nach Anspruch 19, dadurch gekennzeichnet, daß die Schneiden (108a) um den Querschnitt des Werkzeugkörpers (10a) asymmetrisch, insbesondere z.B. 0,5 bis $3^0$ unterschiedliche Winkel einschließend, verteilt sind.

21. Werkzeug nach Anspruch 19 oder 20, dadurch gekennzeichnet, daß das distale Ende des Werkzeug-, insbesondere Fräskörpers (10a) bzw. dessen Fortsatz (11a) vorzugsweise der dortige Bereich der Schneiden (108a), im wesentlichen abgerundet ausgebildet ist.

0209516

22. Werkzeug nach einem der Ansprüche 19 bis 21, dadadurch gekennzeichnet, daß der Werkzeug-, insbesondere
Fräs-Körper (10a) an seinem metaphysären Ende einen im
wesentlichen zylindrischen Ansatz (15a) mit, vorzugsweise die Schneiden (108a) des Fräskörpers (10a) fortsetzenden, Schneiden aufweist.

1/1                    0209516

Fig.1    Fig.3

Fig.1a

Fig.1b

Fig.1c

Fig.2